# EUROPEAN PATENT SPECIFICATION

(11) **EP 2 477 973 B1**
(45) Date of publication and mention of the grant of the patent: **27.08.2014**
(21) Application number: 10760094.2
(22) Date of filing: 16.09.2010
(51) Int. Cl.: C07D 401/04

(54) **PROCESS FOR THE PREPARATION OF A CRYSTALLINE FORM OF LENALIDOMIDE**
VERFAHREN ZUR HERSTELLUNG EINER KRISTALLINEN FORM VON LENALIDOMID
PROCÉDÉ DE PRÉPARATION D'UNE FORME CRISTALLINE DU LÉNALIDOMIDE

(30) Priority: 16.09.2009 IN DE19302009
(43) Date of publication of application: 25.07.2012
(73) Proprietor: Ranbaxy Laboratories Limited, Delhi 110019 (IN)
(72) Inventor: KUMAR, Saridi, Madhava, Dileep, 122101 Gurgaon, Haryana (IN); KAPOOR, Munish, Punjab 143006 (IN); SATHYANARAYANA, Swargam, Karim Nagar Andhra Pradesh 505002 (IN); THAPER, Rajesh, Kumar, Jammu Jammu and Kasmir 180001 (IN); PRASAD, Mohan, Gurgaon Haryana 122001 (IN)
(74) Representative: Cronin, Brian Harold John
(86) International application number: PCT/IB2010/054187
(87) International publication number: WO 2011/033468

(56) References cited:
- WO-A2-2005/023192
- IN-A- 47C HE2 006
- US-A1- 2006 052 609
- US-B1- 6 281 230

## Description

### Field of the Invention

The present invention relates to a process for the preparation of polymorphic Form A of lenalidomide which involves *in-situ* crystallization.

### Background of the Invention

Lenalidomide is an immunomodulatory agent with antiangiogenic and antineoplastic properties. Lenalidomide is indicated for the treatment of myelodysplastic syndromes and for the treatment of multiple myeloma. Lenalidomide is chemically 3-(4-amino-1-oxo-1,3-dihydro-2*H-*isoindol-2-yl)piperidine-2,6-dione of Formula I.

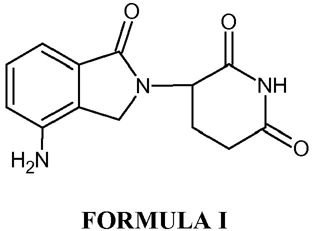

WO 2005/023192 describes polymorphic Forms A, B, C, D, E, F, G and H of lenalidomide. WO 2005/023192 says that Form A can be obtained from various solvents including 1-butanol, butyl acetate, ethanol, ethyl acetate, methanol, methyl ethyl ketone and tetrahydrofuran and provides XRPD, TGA, DSC, IR and Raman data on Form A. Form A is described to be an unsolvated. However, there is no specific method for the preparation of Form A provided in available literature, including WO 2005/023192, which simply mentions that Form A can be prepared by recrystallization from several solvents.

### Summary of the Invention

The present inventors have developed an *in-situ* crystallization process to obtain polymorphic Form A of lenalidomide without the need for isolating crude lenalidomide. The present process is simple and economical and it consistently provides polymorphic Form A of lenalidomide.

### Brief Description of the Drawings

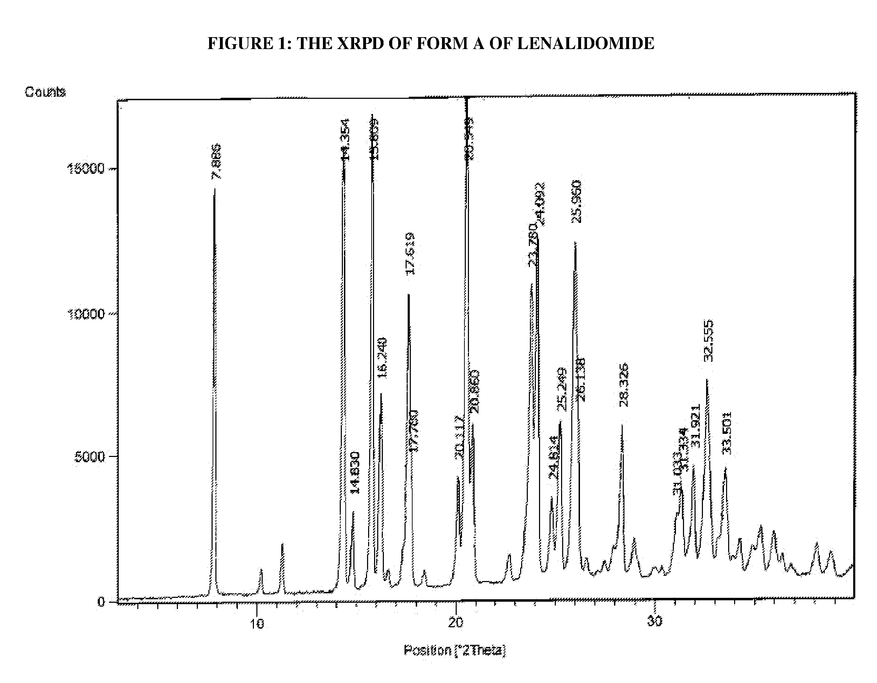

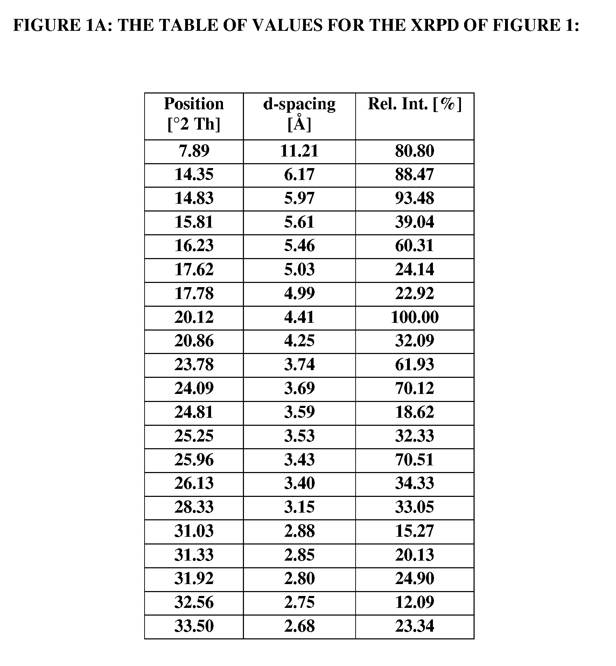

Figure 1 depicts the X-Ray Powder Diffractogram (XRPD) of Form A of lenalidomide.
Figure 1A provides the table of values for the XRPD of Figure 1.

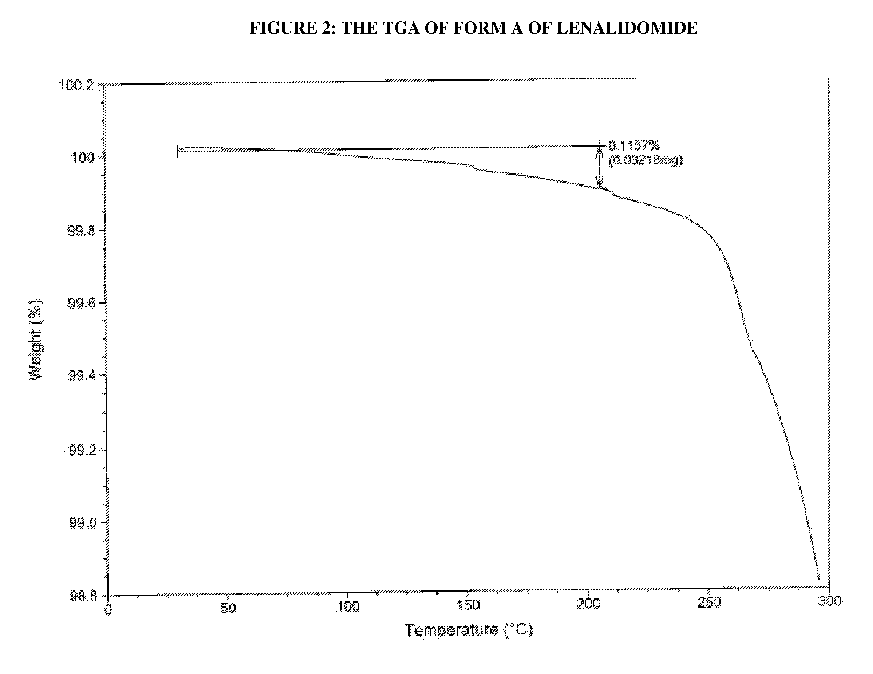

Figure 2 depicts the Thermogravimetric Analysis (TGA) of Form A of lenalidomide.

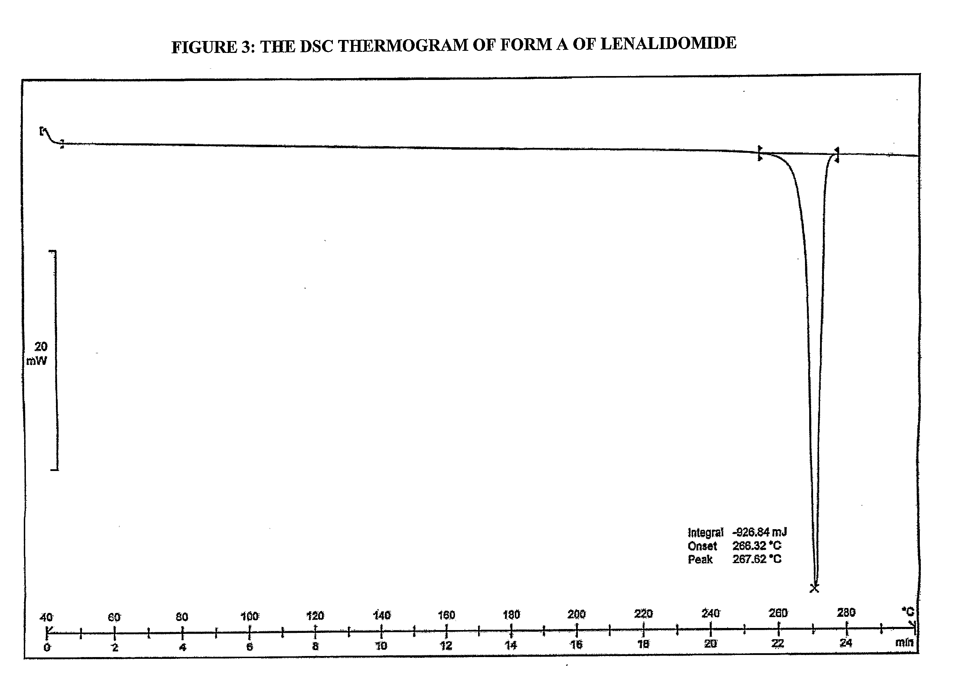

Figure 3 depicts the Differential Scanning Calorimetry (DSC) thermogram of Form A of lenalidomide.

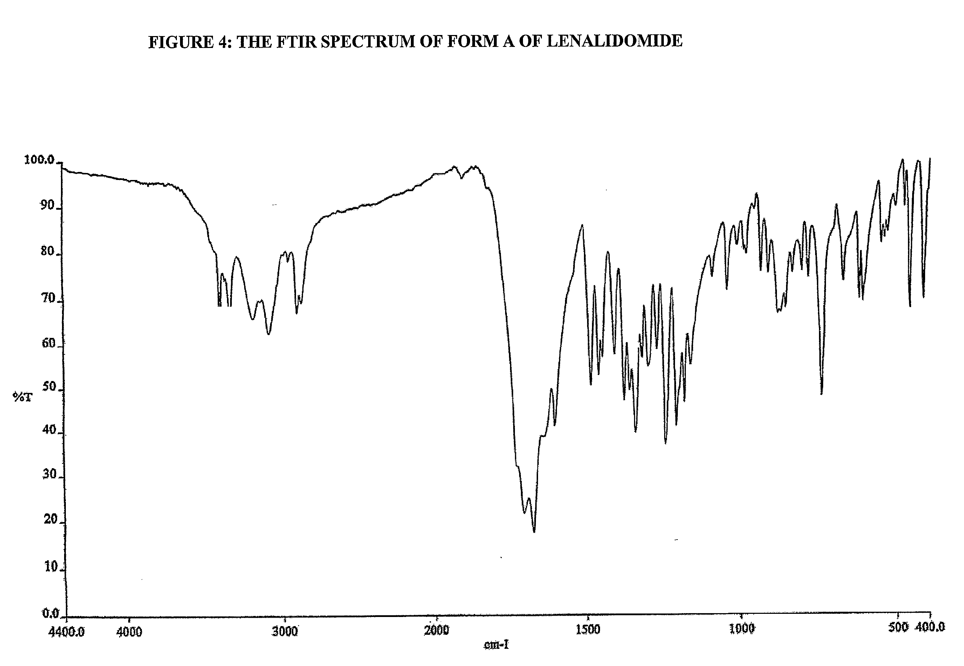

Figure 4 depicts the Fourier-Transform Infra-red (FTIR) spectrum of Form A of lenalidomide.

### Detailed Description of the Invention

The present invention provides a process for the preparation of polymorphic Form A of lenalidomide, wherein the process comprises:
a) reducing 1-oxo-2-(2,6-dioxopiperidin-3-yl)-4-nitroisoindoline in N, N-dimethylformamide to obtain lenalidomide;
b) evaporating off the solvent from step a) and treating the residue with a suitable solvent selected from methyl acetate or 2-methoxy ethanol at a temperature range of 20°C to 60°C for methyl acetate and 70°C to 100°C for 2-methoxy ethanol, followed by stirring at 15°C to 30°C;
c) isolating polymorphic Form A of lenalidomide from the reaction mixture thereof;
wherein, in step b) treatment with methyl acetate is carried out for 1 hour to 50 hours.

The 1-oxo-2-(2,6-dioxopiperidin-3-yl)-4-nitroisoindoline may be prepared according to the methods provided in U.S. Patent No. 5,635,517. Reduction of 1-oxo-2-(2,6-dioxopiperidin-3-yl)-4-nitroisoindoline may be carried out using a reducing agent, for example, palladium-carbon in the presence of hydrogen atmosphere and N,N-dimethylformamide. The palladium-carbon may be about 10% to about 70% wet, for example, from about 30% to about 60% wet. The temperature of the reaction may be maintained from 20°C to 60°C, for example, from about 30°C to about 40°C. The hydrogen pressure in hydrogenator may be maintained from about 40 psi to about 70 psi (about 275.8 to 482.6 kPa). After the completion of reduction, the reaction mixture may be filtered to remove the catalyst.

The reaction mixture may be concentrated by removing N, N-dimethylformamide, for example, to obtain a solid or semisolid, prior to treatment with the suitable solvent.

The treatment with methyl acetate may be carried out at a temperature from 20°C to 60°C, for example, from about 40°C to about 55°C for 1 hour to 50 hours. The formation of Form A may be effected by stirring the mixture. Form A is isolated from reaction mixture by filtration, concentration, decantation, or a combination thereof.

The treatment with 2-methoxyethanol may be carried out by preparing a solution comprising lenalidomide in 2-methoxyethanol, for example by heating to 70°C to 100°C, followed by stirring at 15°C to 30°C. Form A is isolated from the reaction mixture by filtration, concentration, decantation, or a combination thereof.

XRPD of the samples were determined using X-Ray diffractometer, Rigaku Corporation, RU-H3R, Goniometer CN2155A3, X-Ray tube with Cu target anode, Power: 40 KV, 100 Ma, Scanning speed: 2 deg/min step: 0.02 deg, Wave length: 1.5406 Å.

FTIR spectra of the samples were recorded on a Perkin-Elmer 16 PC instrument, as potassium bromide pellets.

The TGA was recorded on TA (Q500) (Rate of heating = 10°C/minute).

The DSC was recorded on Mettler Toledo (DSC 821) (Rate of heating = 10°C/minute).

While the present invention has been described in terms of its specific embodiments, certain modifications and equivalents will be apparent to those skilled in the art and are intended to be included within the scope of the present claims.

### Example 1: Preparation of Form A of Lenalidomide:

The 1-oxo-2-(2,6-dioxopiperidin-3-yl)-4-nitroisoindoline (40 g), N, N-dimethyl formamide (500 ml) and 50% wet 10% palladium-carbon (4 g) were charged in a hydrogenator. The hydrogen pressure was maintained in the hydrogenator at 50 psi to 60 psi for 3 hours. The reaction mixture was subsequently filtered through a Celite bed and washed with N, N-dimethylformamide (100 ml). The N, N-dimethylformamide was recovered from the filtrate under vacuum at 65°C to 70°C to obtain a solid. Methyl acetate (200 ml) was added to the solid and the mixture was warmed to 45°C to 50°C. The reaction mixture was stirred for 4 hours at 45°C to 50°C, filtered, washed with methyl acetate (50 ml) and dried under vacuum at 45°C to 50°C to obtain the title compound.
Yield: 33.86 g

### Example 2: Preparation of Form A of Lenalidomide

The 1-oxo-2-(2,6-dioxopiperidin-3-yl)-4-nitroisoindoline (40 g), N, N-dimethylformamide (500 ml) and 50% wet 10% palladium-carbon (4 g) were charged in the hydrogenator. The hydrogen pressure was maintained in the hydrogenator at 50 psi to 60 psi (344.7 to 413.7 kPa) for 3 hours. The reaction mixture was subsequently filtered through a Celite bed and washed with N, N-dimethylformamide (50 ml). N, N-dimethylformamide was recovered from the filtrate under vacuum at 65°C to 70°C to obtain a solid. The 2-methoxyethanol (100 ml) was added to the solid and the mixture was warmed to 80°C. The reaction mixture was stirred for 3 hours at 80°C to obtain a clear solution. The reaction mixture was cooled to 20°C to 25°C and stirred for 3 hours further. The mixture was filtered and dried under vacuum at 50°C to 55°C to obtain the title compound.
Yield: 9.0 g

## Claims

1. A process for the preparation of polymorphic Form A of lenalidomide corresponding to the representative X-ray powder diffraction pattern provided in Figure 1, wherein the process comprises:
a) reducing 1-oxo-2-(2,6-dioxopiperidin-3-yl)-4-nitroisoindoline in N, N-dimethylformamide to obtain lenalidomide;
b) evaporating off the solvent from step a) and treating the residue with a suitable solvent selected from methyl acetate or 2-methoxy ethanol at a temperature range of 20°C to 60°C for methyl acetate and 70°C to 100°C for 2-methoxy ethanol, followed by stirring at 15°C to 30°C;
c) isolating polymorphic Form A of lenalidomide from the reaction mixture thereof;
wherein, in step b) treatment with methyl acetate is carried out for 1 hour to 50 hours.

2. A process according to claim 1, wherein step a) further comprises concentrating the reaction mixture by removing N, N-dimethylformamide.

3. A process according to claim 1, wherein polymorphic Form A of lenalidomide is isolated from the reaction mixture by filtration, concentration, decantation; or a combination thereof.

4. A process according to claim 1, for the preparation of polymorphic Form A of lenalidomide, wherein step b) of the process comprises evaporating off the solvent from step a) and treating the residue with methyl acetate, wherein this treatment is carried out at a temperature in the range of 20°C to 60°C.

5. A process according to claim 4, wherein step b) further comprises heating the reaction mixture at 40°C to 55°C and stirring at same temperature.

6. A process according to claim 1, for the preparation of polymorphic Form A of lenalidomide, wherein step b) of the process comprises evaporating off the solvent from step a) and treating the residue with 2-methoxyethanol, wherein this treatment is carried out at a temperature in the range of 70°C to 100°C.

7. A process according to claim 6, wherein step b) further comprises heating the reaction mixture at 70°C to 100°C and stirring at 15°C to 30°C.

## Patentansprüche

1. Verfahren zur Herstellung der polymorphen Form A von Lenalidomid entsprechend dem in Figur 1 angegebenen typischen Röntgenpulverdiffraktogramm, wobei das Verfahren umfasst
a) Reduzieren von 1-Oxo-2-(2,6-dioxopiperidin-3-yl)-4-nitroisoindolin in N,N-Dimethylformamid zur Gewinnung von Lenalidomid;
b)Abdampfen des Lösungsmittels aus Schritt a) und Behandeln des Rückstandes mit einem geeigneten Lösungsmittel, ausgewählt aus Methylacetat oder 2-Methoxyethanol in einem Temperaturbereich von 20 °C bis 60 °C für Methylacetat und 70 °C bis 100 °C für 2-Methoxyethanol und danach Rühren bei 15 °C bis 30 °C;
c) Abtrennen der polymorphen Form A von Lenalidomid aus diesem Reaktionsgemisch;
wobei im Schritt b) die Behandlung mit Methylacetat über 1 Stunde bis 50 Stunden ausgeführt wird.

2. Verfahren nach Anspruch 1, wobei der Schritt a) ferner das Einengen des Reaktionsgemisches durch Entfernen von N, N-Dimethylformamid umfasst.

3. Verfahren nach Anspruch 1, wobei die polymorphe Form A von Lenalidomid aus dem Reaktionsgemisch durch Filtrieren, Einengen, Dekantieren oder eine Kombination aus diesen abgetrennt wird.

4. Verfahren nach Anspruch 1 zur Herstellung der polymorphen Form A von Lenalidomid, wobei der Schritt b) des Verfahrens das Abdampfen des Lösungsmittels aus Schritt a) und die Behandlung des Rückstands mit Methylacetat umfasst, wobei diese Behandlung in einem Temperaturbereich von 20 °C bis 60 °C ausgeführt wird.

5. Verfahren nach Anspruch 4, wobei der Schritt b) ferner das Erwärmen des Reaktionsgemisches auf 40 °C bis 55 °C und das Rühren bei derselben Temperatur umfasst.

6. Verfahren nach Anspruch 1 zur Herstellung der polymorphen Form A von Lenalidomid, wobei der Schritt b) des Verfahrens das Abdampfen des Lösungsmittels aus
Schritt a) und die Behandlung des Rückstandes mit 2-Methoxyethanol umfasst, wobei diese Behandlung bei einer Temperatur im Bereich von 70 °C bis 100 °C ausgeführt wird.

7. Verfahren nach Anspruch 6, wobei der Schritt b) ferner das Erwärmen des Reaktionsgemisches auf 70 °C bis 100 °C und das Rühren bei 15 °C bis 30 °C umfasst.

## Revendications

1. Procédé pour la préparation de la forme polymorphe A du lénalidomide correspondant au modèle représentatif de diffraction des rayons X sur poudre indiqué sur la figure 1, le procédé comprenant :
a) la réduction de 1-oxo-2-(2,6-dioxopipéridin-3-yl)-4-nitroisoindolidine en N,N-diméthylformamide pour obtenir du lénalidomide ;
b) l'évaporation du solvant de l'étape a) et le traitement du résidu avec un solvant adéquat sélectionné parmi l'acétate de méthyle ou le 2-méthoxyéthanol dans une plage de température de 20 °C à 60 °C pour l'acétate de méthyle et de 70 °C à 100 °C pour le 2-méthoxyéthanol, suivie par une agitation entre 15 °C et 30 °C ;
c) l'isolement de la forme polymorphe A du lénalidomide à partir de son mélange réactionnel ;
dans lequel, à l'étape b) un traitement à l'acétate de méthyle est réalisé pendant 1 heure à 50 heures.

2. Procédé selon la revendication 1, dans lequel l'étape a) comprend en outre la concentration du mélange réactionnel par l'élimination du N,N-diméthylformamide.

3. Procédé selon la revendication 1, dans lequel la forme polymorphe A du lénalidomide est isolée du mélange réactionnel par filtration, concentration, décantation ; ou une combinaison de celles-ci.

4. Procédé selon la revendication 1, pour la préparation de la forme polymorphe A du lénalidomide, dans lequel l'étape b) du procédé comprend l'évaporation du solvant de l'étape a) et le traitement du résidu à l'acétate de méthyle, ce traitement étant réalisé à une température dans la plage de 20 °C à 60 °C.

5. Procédé selon la revendication 4, dans lequel l'étape b) comprend en outre le chauffage du mélange réactionnel entre 40 °C et 55 °C et l'agitation à la même température.

6. Procédé selon la revendication 1, pour la préparation de la forme polymorphe A du lénalidomide, dans lequel l'étape b) du procédé comprend l'évaporation du solvant de l'étape a) et le traitement du résidu au 2-méthoxyéthanol, ce traitement étant réalisé à une température dans la plage de 70 °C à 100 °C.

7. Procédé selon la revendication 6, dans lequel l'étape b) comprend en outre le chauffage du mélange réactionnel entre 70 °C et 100 °C et l'agitation entre 15 °C et 30 °C.
